# EUROPEAN PATENT APPLICATION

(11) **EP 1 375 634 A1**
(43) Date of publication of application: **02.01.2004**
(21) Application number: 02077540.9
(22) Date of filing: 26.06.2002
(51) Int. Cl.: C11D 3/39, A01N 37/16, A01N 59/00

(54) **Aqueous disinfecting compositions with rapid bactericidal effect**

(71) Applicant: Tevan B.V., 4207 HE Gorinchem-Oost (NL)
(72) Inventor: van Oosterom, Piet J.A., 3544 WD Utrecht (NL)
(74) Representative: Bird, William Edward

(57) **Abstract**

An aqueous disinfecting composition based on hydrogen peroxide and a mixture of carboxylic and percarboxylic acids, wherein the weight ration of the said mixture to hydrogen peroxide is from 0.002 to 0.08, and wherein the said composition further comprises an effective amount of a metal component as a source of metal ions, as well as the use of said aqueous disinfecting composition for cleaning, decontaminating, disinfecting or sterilizing a solid surface or a volume of a gas or liquid.

## Description

The present invention relates to aqueous disinfecting, decontaminating, depolluting and cleaning compositions. It further relates to a method for their preparation and to various uses of the said compositions on the one hand, in the field of hygiene, for cleaning surfaces and on the other hand in the field of waste water treatment and pollution management.

### BACKGROUND OF THE INVENTION

Effective disinfecting processes are necessary for the treatment of bulky amounts of water, especially domestic and industrial circulating waters, and aqueous effluents (such as being present in the foodstuff processing industry) containing microorganisms which cannot be discharged or re-used untreated for hygienic, operational or environmental reasons. Effective disinfecting processes are also necessary for treating surfaces such as premises, equipment, containers, air-conditioning systems and the like. Environmentally compatible disinfecting processes are mainly based on the use of active oxygen compounds, such as hydrogen peroxide and/or lower percarboxylic acids, especially peracetic acid. Other technical solutions involve an aqueous composition containing hydrogen peroxide and silver in the form of a salt or complex.

Hydrogen peroxide is a moderately active, mild disinfectant with bactericidal properties. Hydrogen peroxide concentrations of 25 mg/l are known to inhibit the growth of some bacteria, however an effective reduction of the germ count, even at a much higher hydrogen peroxide concentration, takes many hours or requires additional ultraviolet radiation. Generation of the latter, however, requires both expensive equipment and substantial electricity costs. Therefore when disinfecting large amounts of water, for instance for the treatment of water in sewage works and their outputs, such measures are practically inadequate and/or uneconomic.

Peracetic acid is a highly effective disinfectant used to effect rapid germ reduction. Accordingly, peracetic acid is used for sterilization in the food industry and for disinfecting bottles and equipment in hospitals.

It is well known in the art that a lower aliphatic percarboxylic acid having the formula RCO₃H, wherein R is methyl or ethyl, may be prepared according to the equilibrium reaction represented by equation (1): 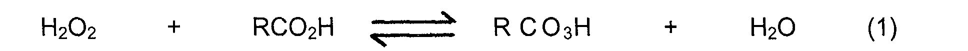 and that, in order to stabilize concentrated aqueous solutions of such a percarboxylic acid, it is recommended to incorporate hydrogen peroxide into said compositions.

Thus, due to the above-mentioned process of manufacture of peracetic acid (i.e. wherein R is methyl) solutions, the latter contain not only peracetic acid and water but also hydrogen peroxide and acetic acid, most often together with one or more stabilizers. Commercially available peracetic acid (PAA) solutions contain (by weight) 15% PAA, 14% hydrogen peroxide and 28% acetic acid or, respectively, 38% PAA, 4 % hydrogen peroxide and 44.7% acetic acid. Because of their corrosive and fire-promoting properties, the use of such highly concentrated peracetic acid solutions creates many handling, storage and transport problems. Of particular concern is the odor of peracetic acid and its irritating properties with respect to the skin, eyes, and respiratory tract. Another problem, as far as the disinfection of food industry equipment is concerned, is the use of stainless steel. When, for economic purposes, deionized water is replaced with water containing high levels of dissolved chlorides (for example water which was treated with chlorine-containing products) for the dilution of peracetic acid solutions, then insidious corrosion of the stainless steel equipment may occur. Disproportionately high acetic acid content is also responsible for increasing the chemical oxygen demand of water to be disinfected. Yet another problem is that this extremely high proportion of free acetic acid (additional acetic acid being formed from the decomposition of peracetic acid) forms the basis for a renewed and possibly explosive proliferation of the germs.

The following forms a review of prior art pertaining to the field of the present invention. U.S. Patent No. 4,051,059 discloses silver-free microbicides stable in storage comprising (by weight) 0.5 to 20% of an acetic/peracetic acid mixture, 25 to 40% hydrogen peroxide and the remainder to 100% water. Acetic acids mixture/H₂O₂ weight ratios from 0.166 to 0.866 are disclosed in examples 1-2 and 4-5. Similar acetic acids mixture/H₂O₂ weight ratios from 0.15 to 0.18 are also disclosed in the examples of U.S. Patent No. 4,051,058 relating to similar silver-free microbicides. Table II of the latter document shows microbial efficiencies (measured as log reductions of germs) after 5 minutes of 5.3 for *Eschericia.coli* and 4.9 for *Pseudomonas aeruginosa*.

U.S. Patent No. 4,743,447 discloses disinfecting contact lenses with a silver-free, stable, ready-to-use composition containing (by weight) 0.005 to 0.1% peracetic acid, 1 to 8% hydrogen peroxide and sufficient acetic acid for the system to reach equilibrium. According to the examples, this means acetic acids mixture/H₂O₂ weight ratios from 0.161 to 0.513. This document further teaches microbial efficiencies (measured as log reductions of germs) after 5 minutes of 5.0 for *Pseudomonas aeruginosa, Streptococus faecalix, Candida albicans* and *Klebsiella pneumoniae.*

In its single example, U.S. Patent No. 4,587,264 discloses a silver-free disinfecting composition with an acetic acids mixture/H₂O₂ weight ratio of 0.41.

U.S. Patent No. 6,277,414 discloses a decontaminating composition comprising a mixture of H₂O₂, peracetic acid, acetic acid, silver (in the form of a salt or complex as a source of Ag⁺ ions) and a stabilizer (such as H₃PO₄, also acting as a pH regulator), wherein the four components H₂O₂, peracetic acid, acetic acid and Ag⁺ have a synergistic effect in respect of decontamination. More specifically, the composition has an H₂O₂ content not above 60% (preferably not above 8% from the standpoint of safe transport regulations), an acetic+peracetic acid mixture/H₂O₂ weight ratio of 0.15:1 to 0.85:1 and a silver component/H₂O₂ weight ratio between 0.0005 and 0.015, and comprises an amount of water to make up to 100% by weight, whereas the respective amounts of acetic and peracetic acids in the mixture are not critical. However, methods making use of the germicidal effect of silver ions are known from GB-A-2,189,394 to be slow-working. This is further confirmed by the experiments of U.S. Patent No. 6,277,414 wherein log reduction of germicidal or sporicidal activity is measured after an exposure time of the support of 2 hours.

Thus, there is a strong consensus in the art that a disinfecting composition based on hydrogen peroxide, acetic acid and peracetic acid, whether free of metal ions or not, should exhibit an acetic acids mixture/H₂O₂ weight ratio of at least 0.15. There is also a prejudice in the art against the use silver ions because of their slow efficiency against germs.

A few other documents, such as for instance EP-A-357,598 and EP-A-517,742, even suggest acetic acids mixture/H₂O₂ weight ratios above 1. For instance, EP-A-357,598 discloses a stable microbicide comprising from 0.2 to 8% hydrogen peroxide, from 0.2 to 11% of a mixture of peracetic acid and acetic acid, and the remainder of 100% water, wherein the ratio acids/ H₂O₂ is between 1 and 11 and the acetic acid/peracetic acid ratio is from about 13 to 100. EP-A-517,742 discloses the problematic of aqueous dilute solutions of peracetic acid being produced by reacting concentrated hydrogen peroxide and acetic acid and being equilibrium mixtures of the reactants and the reaction products. Under relatively forcing conditions, a concentrated mixture may reach equilibrium within hours, however when a concentrated equilibrium solution is diluted with water, the equilibrium point of the system is altered in favour of the regeneration of the original reactants and the ageing time required to reach the new equilibrium after dilution is similar, i.e. a month or more, to that required to directly produce a dilute solution. Based upon this statement, EP-A-517,742 provides an improved process for making a diluted solution containing from 0.5 to 15% of peracetic acid. For use in industrial or domestic disinfection, said solution may be further diluted until an effective peracetic acid content of about 0.1 to 2%.

On the other hand, two documents suggest acetic acids mixture/H₂O₂ weight ratios below 0.15 under very specific conditions. For instance, EP-A-678,123 discloses disinfecting an aqueous solution containing a substantial concentration of nutrients for bacteria, especially the pressed pulp water recycle of a sucrose extracting plant, by using a biocide made by reacting a concentrated hydrogen peroxide solution (i.e. containing at least 30% H₂O₂) with a minor amount of acetic acid, the mole ratio of hydrogen peroxide to acetic acid being about 10:1 to 30:1 (i.e. an acetic acids/H₂O₂ weight ratio from 0.06 to 0.18), and thereafter permitting the mixture to reach equilibrium. Example 1 indicates a log reduction of germs of 3 with a peracetic acid/H₂O₂ weight ratio of 0.1. No source of metal ions is used in the aqueous disinfectant of this document.

U.S. Patent No. 5,965,033 teaches improving the long term action of a disinfectant for sewage works outputs and industrial circulating waters by using a disinfectant with a hydrogen peroxide weight content of 40-60% for retarding germ growth, a peracetic acid weight content of 1.5-2.5% and an acetic acid weight content to 0.2-2%, i.e. acetic acids mixture/H₂O₂ weight ratios from about 0.03 to 0.07. No source of metal ions is used in the disinfectant composition of this document. The single example indicates a log reduction of germs of only 2 after an exposure time of 3 hours to a disinfectant containing 6 ppm peracetic acid and 150 ppm hydrogen peroxide.

Prior art in the field also includes EP-A-0 370 850 disclosing an aqueous solution comprising (by weight) 6 to 8% hydrogen peroxide, 0.1 to 1% peracetic acid and 2 to 10% acetic acid as a hygiene agent for disinfecting hemodialysis equipment.

EP-A-678,123 by principle exclude any dilution of the disinfecting composition and requires safety precautions for the storage of concentrated hydrogen peroxide solutions. It is well known, namely from US-A-6,277,414, that such concentrated solutions exhibit difficulties as regards transportation when the hydrogen peroxide content is above 16% or 8% depending on the national regulations which require special vented packaging for restricting transport. Also, a comparison of the germ reductions achieved by prior art with acetic acids mixture/H₂O₂ weight ratios below 0.15, i.e. EP-A-678,123 and US-A-5,965,033, with those obtained by formulations with acetic acids mixture/H₂O₂ weight ratios above 0.15, including US-A-6,277,414, clearly shows a dramatic decrease of the disinfecting efficiency and speed. Thus, there is a strong prejudice in the art of decontaminating and cleaning formulations against any attempt to reduce the amounts of acetic and peracetic acid versus the amount of hydrogen peroxide.

Thus, there is a strong need in the art for aqueous disinfecting solutions based on hydrogen peroxide and lower aliphatic carboxylic acids and the corresponding carboxylic peracids (preferably mixtures of acetic acid and peracetic acid), being able to provide a high disinfecting efficiency, especially as conventionally measured by reduction of germs, for a wide range of germs (including bacteria, fungi, viruses and the like), within a short period of time, without a significant risk of renewed proliferation of germs and while achieving a satisfactory compromise of environmental and safety requirements, such as minimizing odor and irritation of the human skin or mucosa. There is also a need in the art for disinfecting solutions based on hydrogen peroxide and the aforesaid carboxylic acids which meet all current safety national regulations applicable to the handling of the ingredients of such formulations, i.e. hydrogen peroxide and percarboxylic acids at each step of their preparation or use, for instance during manufacturing, storage or transportation. There is also a need in the art for such disinfecting solutions which, upon demand, can be readily used either for the decontamination of bulky amounts of waste water or, following mere dilution with water, for the cleaning, decontamination or sterilization of surfaces such as hospital, laboratory, domestic and industrial premises and equipment, including containers, incubators, swimming pools and the like. All these needs constitute the various goals of the present invention.

### SUMMARY OF THE INVENTION

The present invention is based on the quite unexpected finding that, contrary to several prejudices well-established in the art, the various needs as herein-above identified, can be met simultaneously by aqueous disinfecting compositions based on hydrogen peroxide and a mixture of lower aliphatic carboxylic and percarboxylic acids, provided that the weight ratio of the said mixture to hydrogen peroxide in the said composition is significantly lowered, for instance within a range from about 0.002 to about 0.08, and that the said composition further comprises a suitable amount of a metal component as a source of metal ions.

### DETAILED DESCRIPTION OF THE INVENTION

A first aspect of the present invention therefore consists of an aqueous disinfecting composition comprising, by weight:
(a) from about 0.1 to about 55% of hydrogen peroxide (H₂O₂),
(b) from about 0.01 to about 4% of an RCO₃H/RCO₂H mixture, wherein R is methyl or ethyl,
(c) from about 0.0004 to about 0.5% of a metal component as a source of metal ions, and
(d) an amount of water to make up to 100%,
characterized in that the weight ratio of the RCO₃H/RCO₂H mixture to hydrogen peroxide (H₂O₂) in the said aqueous disinfecting composition is from about 0.002 to about 0.08.

The respective amounts of RCO₃H and RCO₂H in the RCO₃H/RCO₂H mixture being present as component (b) of the aqueous disinfecting composition are not especially critical in the present invention. Given general knowledge available to those skilled in the art, in particular the above-mentioned equilibrium reaction (1), a suitable mixture of (a) and (b), i..e. a ternary mixture of hydrogen peroxide (H₂O₂), lower aliphatic carboxylic acid RCO₂H and corresponding carboxylic peracid RCO₃H is obtainable in having either H₂O and RCO₃H or H₂O₂ and RCO₂H together in H₂O, provided that hydrogen peroxide is in the prerequisite excess relative to the RCO₃H/RCO₂H mixture. Parameters such as time, temperature and concentration which are suitable to influence, if desired for a specific purpose, the RCO₃H/RCO₂H molar ratio in the RCO₃H/RCO₂H mixture are well known to the skilled person. Using commercially available lower aliphatic carboxylic peracid solutions or modifying the latter by further appropriate dilution and/or storage time will usually result in suitable RCO₃H/RCO₂H mixtures wherein the RCO₃H/RCO₂H molar ratio is preferably in the range from about 1:4 to about 5:1, more preferably in the range from about 1:1 to about 4:1. A mixture wherein such molar ratio is below about 1:4 is likely to provide insufficient disinfecting properties in many circumstances, whereas it may be difficult to keep and maintain a mixture with such a molar ratio above about 5:1 whatever the existing temperature, concentration and time circumstances prevailing at the period of use.

In the present aqueous disinfecting compositions, in order to achieve the above-mentioned goals and the unexpected technical effects of this invention whatever the hydrogen peroxide content in a range from about 0.1 to about 55% by weight (including diluted compositions with a preferred hydrogen peroxide content in a range from about 0.15 to about 8% by weight), it is required a weight ratio of the RCO₃H/RCO₂H mixture to hydrogen peroxide (H₂O₂) from about 0.002 to about 0.08, preferably from 0.004 to 0.07, more preferably from 0.005 to 0.05.

Generally, a lower aliphatic monocarboxylic acid RCO₂H wherein R is methyl (i.e. acetic acid) is preferred to the corresponding acid wherein R is ethyl (i. e. propionic acid), because it is a more active disinfecting means in the aqueous disinfecting compositions according to the invention.

The metal component (c) of the aqueous disinfecting compositions according to this invention is preferably a source of metal ions, more preferably a component of a monovalent metal such as silver. The said silver component preferably is a source of Ag⁺ ions. The metal component (c) may also be a component of a plurivalent metal such as specified below. The metal of component (c) may thus also be selected from rare earth metals and from metals of Groups I (A, B), II (A, B), III A, IV (A, B), V (A, B), VIB, VIIB and VIII of the Periodic Table. For instance, the metal may be selected from the group consisting of Mn, Zn, Sn, Fe, Cu, Al, Ni, Co, Ti, V, Zr, Nb, Ta, Cr, Mo, W, La, Bi, K, Cd, Yb, Dy, Nd, Ce, In, Tl, Pr and combinations thereof. Specific examples of suitable plurivalent metals include Ag²⁺, Ag³⁺ and the like.

Suitable metal components (c) include metal complexes, metal chelates and metal salts with either an organic acid or an inorganic acid. Silver nitrate is especially preferred. Other suitable metal salts may be selected from the group consisting of phosphates, carbonates, aminobenzoates, hydroxides, cinnamates, naphthenates, stearates, caproates, laurates, myristates, palmitates, oleates, picolinates, fluorides, aspartates, gluconates, iodides, oxides, nitrites, nitrates, phosphates, pyrophosphates, sulfides, acetates, ascorbates, chlorides, benzoates, citrates, fumarates, gluconates, glutarates, lactates, maleates, malonates, salicylates, succinates, sulfates, and combinations thereof. When the disinfecting composition of the invention is likely to come in contact with the human skin or mucosa, dermatologically acceptable metal chelates and salts like bishistidine complexes, bromides, chondroitin sulfate, chromites, cyanides, dipiocolinates, ethylhexanoates, glycerolate complexes, methoxides, polyphosphonates, paraphenolsulfonates, perchlorates, phenolsulfonates, selenides, thiocyanates, tripolyphosphates, tungstates and the like may also be used.

The said metal component (c) preferably has high water-solubility, therefore a poorly water-soluble component such as silver oxide Ag₂O is less suitable for the purpose of the present invention because it will require solubilizing means (such as bringing the component in a colloidal form) that will add to the complexity of the composition and its manufacturing process.

The amount of the metal component (c) is between about 0.0004 and about 0.5% by weight of the disinfecting composition, depending upon the hydrogen peroxide content thereof. Preferably the weight ratio of the metal component to the hydrogen peroxide is within the range of about 0.00005:1 to about 0.003:1, more preferably within the range of 0.0005:1 to about 0.002:1.

The aqueous disinfecting compositions according to this invention may further include one or more stabilizers, such as a strong inorganic acid, for instance phosphoric acid, nitric acid, sulfuric acid, hydrobromic acid, boric acid or mixtures thereof. Among inorganic acid stabilizers, phosphoric acid is especially preferred. The stabilizer may also be either gelatin or an organic carboxylic acid such as tartaric acid, citric acid (or a hydrate thereof), benzoic acid, picolinic acid, nicotinic acid and isonicotinic acid. The said stabilizer(s), when present, should preferably be used in an effective amount close to the amount of the metal component.

The aqueous disinfecting compositions according to this invention may also include at least one component selected from the group consisting of surfactants, corrosion inhibitors and fragrances.

Suitable surfactants for use herein include anionic, cationic, non-ionic, amphoteric and zwitterionic surface-active compounds, preferably those suitable for contact with foodstuffs or drinking water at the relevant dose, and mixtures of such compounds.

A wide variety of anionic surfactants are potentially useful herein. Nonlimiting examples of anionic surfactants include those selected from the group consisting of alkyl and alkyl ether sulfates, sulfated monoglycerides, alkylarylsulfonates, primary or secondary alkane sulfonates, alkyl sulfosuccinates, acyl taurates, acyl isothionates, alkyl glycerylether sulfonates, sulfonated methyl esters, sulfonated fatty acids, alkyl phosphates, acyl glutamates, alkyl sulfoacetates, acylated peptides, alkyl ether carboxylates and mixtures thereof. Exemplary embodiments include alkylbenzenesulfonates having 6 to 18 carbon atoms in the alkyl group, alkylsulfates and alkanesulfonates (each having 8 to 22 carbon atoms in the alkyl or alkane group) of alkaline earth metals and alkali metals (particularly sodium or potassium), as well as polyethoxylated phosphoric acid alkyl esters. The alkyl ether sulfates are typically made as condensation products of ethylene oxide and monohydric alcohols (having from about 8 to about 24 carbon atoms) and then sulfated. These alcohols can be derived from fats, e.g., coconut oil or tallow, or can be synthetic. Specific examples of alkyl sulfates which may be used in the compositions are sodium, ammonium, potassium, magnesium or triethanolamine salts of lauryl or myristyl sulfate. Other suitable non-ionic and water-soluble surfactants include for instance polyethoxylated and/or polypropoxylated glycols, C₁₀-C₂₀ fatty acid monoesters, sorbitan monopalmitate and the like. Still other suitable anionic surfactants are the alkyl sulfosuccinates, which include disodium N-octadecylsulfosuccinate, diammonium lauryl sulfosuccinate, tetrasodium N-(1,2-dicarboxyethyl)-N-octadecylsulfosuccinate, diamyl ester of sodium sulfosuccinic acid, dihexyl ester of sodium sulfosuccinic acid and dioctyl esters of sodium sulfosuccinic acid. Quaternary ammonium salt cationic surfactants may be used as well. Specific examples of suitable amphoteric surfactants include sodium 3-dodecylaminopropionate, sodium 3-dodecylaminopropane sulfonate, N-alkyltaurines and betaines.

In the disinfecting compositions according to the invention, the weight ratio of the said surfactant to hydrogen peroxide is preferably at most 0.1.

Suitable corrosion inhibitors for use herein should preferably admissible for contact with foodstuffs and/or for oral administration with drinking water. Exemplary compounds include low molecular weight aliphatic phosphonic acids optionally containing carboxyl groups apart from the phosphonic acid groups. These include for instance aminophosphonic acids (such as dimethylaminomethane diphosphonic acid, aminotri-(methylene phosphonic acid, aminoacetic acid-N-di-(methylene phosphonic acid), 1-amino-1-cyclohexylmethane-1,1-diphosphonic acid, 3-aminopropane-1-hydroxyl-1,1-diphosphonic acid and ethylenediamine-tetra-(methylenephosphonic acid), hydroxyethanediphosphonic acid, phosphonosuccinic acid, 2-phosphonobutane-1,2,4- tricarboxylic acid, as well as their water-soluble salts, particularly the alkali metal salts (such as sodium or potassium), the ammonium salts and the alkanolamine salts (preferably wherein the alkanol has 2 or 3 carbon atoms, such as the mono-, di- or tri-ethanolamine salts) and mixtures thereof. When present, the corrosion inhibitor should preferably be used in a weight ratio, with respect to hydrogen peroxide, not above about 0.03.

The fragrance, when present, should preferably be incorporated into the aqueous disinfecting composition of the invention in an amount of at most about 0.5% by weight of the composition.

Water which forms the main part of the disinfecting composition according to the invention is preferably a purified water, namely a distilled water, demineralized water or deionized water grade. The latter preferably means a water having a resistivity greater than about 10⁵ 'Ω/cm and preferably greater than 10⁶ 'Ω /cm.

The pH of the aqueous compositions according to the invention is preferably between about 1.5 and 5, more preferably between about 2 and 4 and may be regulated, if necessary, by means of phosphoric acid as previously indicated.

The aqueous compositions of this invention may be manufactured according to various methods. In a first embodiment, all ingredients of the composition may be mixed at the same time. However, an alternative embodiment may be preferred, wherein first a stabilized metal component aqueous solution is prepared by mixing the said metal component together with one or more acidic stabilizers (such as above described) and water and then in a second step mixing the stabilized metal component aqueous solution together with hydrogen peroxide (preferably a 40 to 60% concentrated solution) and the lower carboxylic acid RCO₂H or a RCO₃H/RCO₂H mixture. When the metal component is not completely water-soluble, it may be necessary to provide additional solubilization means such as is well known in the art, for instance by adding a small amount of a colloidal agent. In each case, mixing should be effected in a vessel or apparatus made from a material being resistant to hydrogen peroxide, to the acidic stabilizer and to the metal component being a source of metal ions. Suitable but non-limiting examples of such materials include glass, polyethylene and polytetrafluoroethylene. The manufacturing method of the invention is preferably performed under atmospheric pressure and within a temperature range from about 10°C to about 40°C, i.e. there is no need for or advantage resulting from providing specific heating means for raising the temperature above room temperature. The above embodiments of the method are applicable to both concentrated formulations and diluted formulations. However, in the latter case, it is preferred to prepare them by water dilution of the concentrated formulations preferably immediately before use of the same. The water grade to be used for such dilution is not critical to the present invention. It may be a standard hardness water, such as specified in NEN-EN1276 norm (1997), or a purified water grade preferably as indicated herein-above when the added cost of such water grade is consistent with the economic conditions prevailing in the intended specific use of the diluted formulation.

For the sake of clarity, distinction between concentrated formulations and diluted formulations will be based on the hydrogen peroxide content of the said compositions. By definition, a concentrated formulation or composition herein is one including at least 15% by weight and up to about 55% by weight hydrogen peroxide. By contrast, a diluted formulation or composition is defined herein as one including less than 15% by weight hydrogen peroxide.

When an aqueous concentrate composition according to the invention is used as a disinfecting composition, it is typically applied in the following conditions (concentrations and application time):
- when applied to water treatment: at a concentration of preferably at least 50 ml, preferably not more than 30,000 ml, of the said concentrate composition per volume of 1 m³, during at least 24 hours;
- when dispersed into a gas like air, in a concentration of at least about 2 mg, preferably not more than about 2 g of the said concentrate composition per gas volume of 1 m³, during at least about 5 minutes.

When the aqueous disinfecting composition of the invention is applied onto a solid surface, it is preferred for safety regulations to use a diluted formulation obtained by mixing preferably at least 1 % by weight, preferably not more than 15% by weight, of the concentrated composition with water, and moisturizing the said solid surface with the diluted formulation obtained during at least 5 minutes until complete wetting of the solid surface is achieved (which, as is known from the skilled person, may depend upon the surface porosity).

As will be understood to those skilled in the art, the preferred amount of the concentrate composition to be used will widely vary with the type and amount of micro-organisms present on the solid surface or in the liquid or gas to be treated.

With respect to the many uses of the aqueous compositions according to this invention as a disinfectant, the following application methods are more particularly recommended:
- immersion of the product to be treated into the said aqueous composition,
- spraying of the aqueous disinfecting composition onto a solid surface to be treated, and
- incorporation of the aqueous disinfecting (diluted or concentrated) composition into water to be treated (particularly swimming pool water, drinking water, industrial process water, waste water and the like).

Therefore, the aqueous disinfecting compositions according to the invention are especially useful for:
(a) the disinfection and hygiene of hospital and laboratory premises, industrial premises (such as milk dairies, cheese dairies, malt houses, breweries, facilities for the production of mineral water, wine, spirits, fruit and vegetable juices; greenhouses; cowsheds, hen houses and stables; packaging lines for foodstuffs, drinks or pharmaceuticals; interiors of aeroplanes and boats) and the contents of said premises, especially the equipment or instruments within said premises;
(b) the sterilization of aseptic enclosures such as incubators for premature animals or growing axenic animals;
(c) the treatment of legionella in air-conditioning systems;
(d) the disinfection and hygiene of storage containers (especially silos) and pipelines for conveying liquid or solid products such as foodstuffs (sugar, tea, coffee, cereals, drinks) and animal feed;
(e) the disinfection and hygiene of swimming pools and other balneotherapy equipments (in which case the composition will preferably be surfactant-free);
(f) the disinfection of drinking water (for instance in wells or storage containers), in which case the composition will preferably be surfactant-free; and
(g) the protection of outdoor crops (such as cereals, tomatoes, banana plantations, hydroponic cultures including witloof, seeds, tubercules and the like), by virtue of its bactericidal, fungicidal, sporicidal, virucidal and antiparasitic properties.

Thus the present invention encompasses methods of cleaning, decontaminating, disinfecting or otherwise sterilizing a solid surface, a liquid volume or a gaseous volume by applying the aqueous concentrate or diluted composition disclosed above to the said surface or by dispersing it into the said liquid or gas.

The aqueous disinfecting composition of this invention has been found to be active against a wide range of gram-positive and gram-negative bacteria, including for instance *Pseudomonas aeruginosa* (e.g. CMCM-2-22 strain), *Pseudomonas cepacia, Enterobacter cloacae, Enterobacter agglomerans, Klebsiella pneumoniae* (e;g. ATCC-10031 strain), *Eschericia* *coil*, *Streptococcus* faecalis (e.g. ATCC-10541 strain), *Staphylococcus cohnii Staphylococcus aureus* (e.g. IP 52154), (all being customary to hospital bacterial strains), *Enterococcus facium, Enterococcus hirae, Thiobacillus ferrooxidans* (e.g. ATCC 13661 strain), *Lactobacilli, Thermophilic bacilli* and the like. The aqueous disinfecting composition of this invention has also been found to be active against fungi, including for instance *Candida albicans* (e.g. APCC-2091 strain) *Mycobacterium smegmatis* (e.g. IP 7326 strain), *Aspergillus niger* (e.g. 218 IP strain), *Penicillium verrucosum* and the like. The aqueous disinfecting composition of this invention has also been found to have antiparasitic activity against for instance *Schistosoma haematobium,* Schistosoma *mansoni* and the like. The aqueous disinfecting composition of this invention has also been found to have antiviral activity against various viral strains such as for instance orthopoxvirus, human adenovirus, rhinovirus, herpes virus, enterovirus, influenza virus, poliomyelitis virus and the like.

The aqueous disinfecting or decontaminating compositions of the present invention are able to provide bacterial log reductions of at least 5.5, preferably at least 5.9 and more preferably at least 6.1, as measured in accordance with EN1276 norm, after 5 minutes of use in a disinfecting or decontaminating treatment such as mentioned above.

The aqueous disinfecting compositions of the present invention have many advantages over the existing disinfectants, in particular:
- they provide substantial germicide activity within a shorter period of time, within broad temperature limits, namely between about 4°C and 60°C,
- they are ready to use immediately after preparation and can be bottled without further waiting time in customary transport and marketing containers (including those made of glass, metals, porcelain, ceramic, plastics and the like),
- at the preferred concentrations, they exhibit no smell or change in smell with time and produce no health impairment or injury, such as skin irritation, for humans or other mammals, and
- the concentrated formulations can be stored for long periods of time (up to years) at temperatures between about 4°C and 25°C, including incidental temperatures up to about 40°C, without exhibiting a substantial decrease in their germicide activity.

The following examples are provided only for the purpose of illustrating some specific embodiments of the present invention and should not be understood as limiting the scope of the invention in any way.

### EXAMPLE 1 - preparation of a disinfecting concentrate

The following manufacturing steps are performed in a vessel (tank) and with equipment (e.g. stirring apparatus) made of a glass which is resistant to hydrogen peroxide, inorganic and organic acids, and silver ions. The apparatus and equipment is beforehand cleaned well and rinsed with demineralized water with a conductivity below 10 µS/cm. An aqueous concentrate was prepared in two steps at 20°C, under atmospheric pressure and in the absence of ultraviolet light, as follows:

In a first step, 28 g silver nitrate is dissolved into 50 ml demineralized water, then while stirring 10 g tartaric acid is dissolved into the silver nitrate solution thus obtained. Separately, another aqueous solution is prepared by mixing 7 ml demineralized water, 4 g of phosphoric acid (85% pure) and 1 g citric acid monohydrate. While stirring and maintaining the temperature not above 30°C, the latter solution is added to the silver nitrate solution, and the resulting mixture, if not used immediately thereafter in the second step, is stored in the dark.

In the second step, 2.8 g of the stabilized silver concentrate obtained in the first step and stored for at least three days in the dark is added to 1 liter of a mixture comprising (by weight) 1.9% peracetic acid, 47.2% hydrogen peroxide, 0.5% acetic acid and 50.4% water. The resulting mixture is further stirred during 15 minutes and is then ready for bottling or packaging or ready-to-use either as such or after dilution.

The concentrate obtained is stable upon storage for at least 12 months at an average temperature within a range between about 4°C and 25°C, including incidental temperatures of up to about 40°C.

### EXAMPLE 2 - preparation of a disinfecting concentrate

The following manufacturing steps are performed in a vessel (tank) and with equipment (e.g. stirring apparatus) made of a glass which is resistant to hydrogen peroxide, inorganic and organic acids, and silver ions. The apparatus and equipment is beforehand cleaned well and rinsed with demineralized water with a conductivity below 10 µS/cm. An aqueous concentrate was prepared in two steps at 20°C (unless otherwise specified), under atmospheric pressure and in the absence of ultraviolet light, as follows:

In a first step, 31.4 g silver nitrate is dissolved into 0.1 liter demineralized water, then the silver nitrate solution is heated up to 40°C and maintained at this temperature while immersing a redox platinum electrode into the said solution. The redox platinum electrode is connected to a potentiometer. A separately prepared persulfate solution, obtained by dissolving 110 g sodium persulfate into 0.5 liter demineralized water, is added slowly to the silver nitrate solution while stirring. The redox potential controlled by the potentiometer increases immediately after starting addition of the persulfate solution. Said addition is continued slowly until a redox potential of 1,460 mV is attained. The resulting solution is then filtered and kept at a temperature of about 25°C while dissolving 11.2 g tartaric acid therein under stirring. Separately, another aqueous solution is prepared by mixing 7.8 ml demineralized water, 4.4 g of phosphoric acid (85% pure) and 1.12 g citric acid. While stirring and maintaining the temperature not above 30°C, the latter solution is added to the silver solution, and the resulting mixture, if not used immediately thereafter in the second step, is stored for at least three days in the dark.

In the second step, 7.5 ml of the stabilized silver concentrate obtained in the first step and optionally stored in the dark is added to 1 liter of a mixture comprising (by weight) 1.9% peracetic acid, 47.2% hydrogen peroxide, 0.5% acetic acid and 50.4% water. The resulting mixture is further stirred during 15 minutes and is then ready for bottling or packaging or ready-to-use either as such or after dilution.

The concentrate obtained is stable upon storage for at least 12 months at an average temperature within a range between about 4°C and 25°C, including incidental temperatures of up to about 40°C.

### EXAMPLE 3 - preparation of an aqueous disinfecting diluted composition

The following manufacturing steps are performed in a polyethylene vessel (tank) and with equipment (e.g. passivated stainless steel stirring apparatus) which are resistant to hydrogen peroxide, inorganic and organic acids, and silver ions. The apparatus and equipment is beforehand cleaned well and rinsed with demineralized water.

To 755 kg of demineralized water is added 32 kg of the disinfecting concentrate of example 1. After 10 minutes stirring, 80 g of an ethoxy alkylphenol phosphoric ester surfactant (commercially available from ATOFINA) and 160 g of a perfluoroalkylethoxylate phosphate wetting agent (commercially available from DU PONT DE NEMOURS) are added until complete dissolution. A separately prepared mix of 1.4 kg of a perfume (Frenial commercially available from ROBERTET) and 1.2 kg isotridecanol ethoxylate including 8 ethylene oxide units as a surfactant/emulsifier (commercially available from SASOL) is then slowly (i.e. during at least 5 minutes)added to the tank. Finally 4 kg of 2-phosphonobutane-1,2,4-tricarboxylic acid (commercially available from BAYER) is added and stirred during 15 minutes, whereby the aqueous dilute composition thus obtained, having a density of 1.01, is ready-to-use as a disinfecting composition.

### EXAMPLE 4 - Disinfection experiments

The following microbial reduction experiments were performed using conditions in accordance with NEN-EN1276 norm (1997) relating to bacteria and in accordance with the Principles on Good Laboratory Practice as published by OECD, ENV/MC/CHEM/(98)17(1998).

The germicidal activity, measured as the difference, otherwise called log reduction, between the decimal logarithm of the concentration of a certain strain (either bacteria or fungi) before starting treatment and the decimal logarithm of the concentration of the same strain after 5 minutes treatment is shown in the following table 1. Treatment is effected with an aqueous disinfecting diluted composition obtained by mixing 3 volumes of the concentrate of example 1 with 97 volumes of a water grade such as defined in the above-mentioned norm.

**TABLE 1**

| Strain | Log reduction |
|---|---|
| *Candida albicans* (APCC-2091) | 4.7 |
| *Aspergillus niger* | 3.4 |
| *Eschericia coli* | 6.1 |
| *Staphylococcus aureus* | 6.5 |
| *Enterococcus hirae* | 5.5 |
| *Pseudomonas aeruginosa* | 6.3 |

The germicidal activity, otherwise called log reduction, measured after 5 minutes treatment with an aqueous disinfecting diluted composition obtained by mixing 3 volumes of the concentrate of example 2 with 97 volumes of a water grade such as defined in the above-mentioned norm, is shown in the following table 2:

**TABLE 2**

| Strain | Log reduction |
|---|---|
| *Candida albicans* (APCC-2091) | 4.7 |
| *Aspergillus niger* | 3.3 |
| *Eschericia coli* | 6.1 |
| *Staphylococcus aureus* | 6.5 |
| *Enterococcus hirae* | 6.8 |
| *Pseudomonas aeruginosa* | 6.3 |

The germicidal activity measured after 5 minutes treatment with the aqueous disinfecting diluted composition of example 3, otherwise called log reduction, is shown in the following table 3:

**TABLE 3**

| Strain | Log reduction |
|---|---|
| *Eschericia coli* | 5.5 |
| *Staphylococcus aureus* | 6.4 |
| *Enterococcus hirae* | *6.7* |
| *Pseudomonas aeruginosa* | 5.9 |

## Claims

1. An aqueous disinfecting composition comprising by weight:
(a) from 0.1 to 55% of hydrogen peroxide,
(b) from 0.01 to 4% of an RCO₃H/RCO₂H mixture, wherein R is methyl or ethyl, and
(c) from 0.0004 to 0.5% of a metal component as a source of metal ions, and
(d) an amount of water to make up to 100%,
**characterized in that** the weight ratio of the RCO₃H/RCO₂H mixture to hydrogen peroxide in the said composition is from 0.002 to 0.08.

2. An aqueous disinfecting composition according to claim 1, **characterized in that** the RCO₃H/RCO₂H molar ratio in the RCO₃H/RCO₂H mixture is in the range from 1:4 to 5:1.

3. An aqueous disinfecting composition according to claim 1 or claim 2, **characterized in that** the weight ratio of the RCO₃H/RCO₂H mixture to hydrogen peroxide in the said composition is from 0.005 to 0.05.

4. An aqueous disinfecting composition according to any of claims 1 to 3, **characterized in that** the metal of the metal component (c) is a monovalent metal.

5. An aqueous disinfecting composition according to any of claims 1 to 4, **characterized in that** the metal of the metal component (c) is silver.

6. An aqueous disinfecting composition according to any of claims 1 to 5, **characterized in that** the metal of the metal component (c) is a source of Ag⁺ ions.

7. An aqueous disinfecting composition according to any of claims 1 to 6, **characterized in that** the metal component (c) is silver nitrate.

8. An aqueous disinfecting composition according to any of claims 1 to 7, **characterized in that** it further includes one or more stabilizers.

9. An aqueous disinfecting composition according to any of claims 1 to 8, **characterized in that** it further includes one or more corrosion inhibitors.

10. An aqueous disinfecting composition according to any of claims 1 to 9, **characterized in that** it further includes one or more surfactants.

11. An aqueous disinfecting composition according to any of claims 1 to 10, **characterized in that** it further includes one or more fragances.

12. Use of an aqueous disinfecting composition according to any of claims 1 to 11 for cleaning, decontaminating, disinfecting or sterilizing a solid surface or a volume of a gas or liquid.
